# EUROPÄISCHE PATENTSCHRIFT

(11) **EP 1 066 024 B1**
(45) Veröffentlichungstag und Bekanntmachung des Hinweises auf die Patenterteilung: **23.10.2002**
(21) Anmeldenummer: 99913290.5
(22) Anmeldetag: 23.03.1999
(51) Int. Cl.: A61K 7/48, A61K 7/06

(54) **VERFAHREN ZUR HERSTELLUNG VON KOSMETISCHEN REINIGUNGSMITTELN MIT ERHÖHTER VISKOSITÄT**
METHOD FOR PRODUCING HIGH VISCOSITY COSMETIC CLEANSERS
PROCEDE POUR PRODUIRE DES NETTOYANTS COSMETIQUES A VISCOSITE ELEVEE

(30) Priorität: 01.04.1998 DE 19814608
(43) Veröffentlichungstag der Anmeldung: 10.01.2001
(73) Patentinhaber: Cognis Deutschland GmbH & Co. KG, 40589 Düsseldorf (DE)
(72) Erfinder: KAHRE, Jörg, D-42799 Leichlingen (DE); WADLE, Armin, D-40699 Erkrath (DE); SUMSER, Markus, D-44625 Herne (DE)
(86) Internationale Anmeldenummer: EP9901938
(87) Internationale Veröffentlichungsnummer: WO99051199

(56) Entgegenhaltungen:
- WO-A-91/12880
- WO-A-99/09948
- WO-A-99/09949
- WO-A-99/21138
- DE-A- 3 304 897
- DE-A- 19 529 907
- DE-C- 4 338 999
- DE-C- 19 755 491
- GB-A- 1 537 112

## Beschreibung

### Gebiet der Erfindung

Die Erfindung liegt auf dem Gebiet der kosmetischen Zubereitungen und betrifft ein Verfahren zur Kaltherstellung von Haut- und Haarreinigungsmitteln, bei dem man tensidische Fettalkoholdispersionen einsetzt.

### Stand der Technik

Zur Herstellung von Haar- und Hautreinigungsmitteln, wie z.B. Lotionen oder Cremes vom Öl-in-Wasser-Typ, werden zur Einstellung der Viskosität üblicherweise Fettalkohole eingesetzt. Die Herstellung der Emulsionen erfolgt dabei nach dem Heißverfahren, d.h. die Bestandteile werden oberhalb des Schmelzpunktes der am höchsten schmelzenden Komponente vermischt und dann unter intensivem Homogenisieren langsam abgekühlt. Aus Gründen der Wirtschaftlichkeit ist es indes wünschenswert, solche Zubereitungen auf kaltem Wege, beispielsweise unter Einsatz der bekannten PIT- oder Mikroemulsions-Technologie herzustellen. Hierbei tritt jedoch das Problem auf, daß die Einstellung von hohen Viskositäten nur sehr schwer gelingt, insbesondere dann, wenn es darum geht unter Einsatz von Fettalkoholen lamellare Gele herzustellen.

Das Dokument DE 33 04 897 beschreibt ein Verfahren zur Herstellung von kosmetischen Reinigungsmitteln mit erhöhter Viskosität, bei dem man wässrige tensidische Fettalkoholdispersionen mit Ölkomponenten kalt verrührt. In diesem Dokument handelt es sich jedoch nicht um Mikrodispersionen mit Teilchengrösse kleiner 50 µm.

Die Aufgabe der Erfindung hat folglich darin bestanden, ein Verfahren zur Kaltherstellung von kosmetischen Reinigungsmitteln, insbesondere Lotionen und Cremes vom O/W-Typ, zur Verfügung zu stellen, mit dessen Hilfe sich auch lamellare Gele mit besonderer Lagerstabilität herstellen lassen.

### Beschreibung der Erfindung

Gegenstand der Erfindung ist ein Verfahren zur Herstellung von kosmetischen Reinigungsmitteln mit erhöhter Viskosität, bei dem man wäßrige tensidische Fettalkoholdispersionen mit Ölkomponenten kalt verrührt.

Überraschenderweise wurde gefunden, daß sich O/W-Cremezubereitungen für die Reingung und Pflege von Haut und Haaren auch auf kaltem Wege herstellen lassen, wenn man zur Einstellung der Konsistenz tensidische Fettalkoholdispersionen, vorzugsweise Fettalkoholmikrodispersionen einsetzt. Die resultierenden Mittel liegen dabei in Form lamellarer Gele vor, die sich durch eine besonders hohe Stabilität auch bei Temperaturlagerung auszeichnen.

### Fettalkohole

Die im Sinne des erfindungsgemäßen Verfahrens einzusetzenden Dispersionen können Fettalkohole der Formel **(I)** enthalten,

**R**^{**1**}**OH (I)**

in der R¹ für einen linearen oder verzweigten, gesättigten oder ungesättigten Alkylrest mit 6 bis 30 Kohlenstoffatomen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern-, Cetearyl- oder Talgfettalkohol. Der Anteil der Fettalkohole an den Dispersionen beträgt dabei 5 bis 50 und vorzugsweise 25 bis 40 Gew.-%.

### Tenside

Die im Sinne des erfindungsgemäßen Verfahrens einzusetzenden Fettalkoholdispersionen können anionische, nichtionische, kationische und/oder amphotere bzw. zwitterionische Tenside enthalten. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureestersulfate, insbesondere Laurinsäure+1EO-sulfat, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Der Anteil der Tenside an den Fettalkoholdispersionen kann 0,1 bis 10 und vorzugsweise 0,5 bis 5 Gew.-% betragen.

### Fettalkoholdispersionen

Fettalkoholdispersionen sind literaturbekannt. So wird etwa deren Verwendung als Konsistenzverbesserer in **SÖFW-Journal, 121, 216 (1995)** beschrieben. Die Herstellung der Fettalkoholdispersionen kann beispielsweise nach dem Mikrodispersionsverfahren erfolgen. Mikrodispersionen stellen optisch isotrope, thermodynamisch stabile Systeme dar, die eine wasserunlösliche Ölkomponente (hier: Fettalkohol), Dispergatoren - vorzugsweise Alkylglucoside - und Wasser enthalten. Das klare bzw. transparente Aussehen der Mikrodispersionen ist eine Folge der geringen Teilchengröße der dispergierten Emulsionströpfchen. In diesem Zusammenhang hat sich erwiesen, daß Fettalkoholmikrodispersionen einen besonders vorteilhaften Einfluß auf die Herstellung und Lagerstabilität der resultierenden Färbepräparate besitzen. Es werden Dispersionen eingesetzt, die eine Teilchengröße kleiner 50 µm, insbesondere kleiner 20 µm und besonders bevorzugt kleiner 10 µm aufweisen. Übersichten zu Herstellung und Anwendung von Mikrodispersionen finden sich von H.Eicke in **SÖFW-Journal, 118, 311 (1992)** und Th.Förster et al. in **SÖFW-Journal, 122, 746 (1996)**; des weiteren sei auf die Druckschrift **DE-A1 4411557** (Henkel) verwiesen.

### Ölkomponenten

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetytpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### Polymere Verdickungsmittel

In einer weiteren bevorzugten Ausführungsform der Erfindung enthalten die Reinigungsmittel neben den tensidischen Fettalkoholdispersionen polymere Verdickungsmittel, wie z.B. Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon. Die Polymeren können in Mengen von 0,1 bis 5, vorzugsweise 0,5 bis 2 Gew.-% - bezogen auf die Reinigungsmittel - eingesetzt werden.

### Gewerbliche Anwendbarkeit

Unter Einsatz der tensidischen Fettalkoholdispersionen gelingt es auch auf kaltem Wege, d.h. bei Temperaturen im Bereich von 18 bis 25°C kosmetische Reinigungsmittel herzustellen, die in Form lamellarer Gele vorliegen. Als Konsistenzgeber zur Herstellung von kosmetischen Reinigungsmitteln, vorzugsweise von O/W-Lotionen und -Cremes, eignen sich insbesondere Fettalkoholmikrodispersionen, die in den Zubereitungen in Mengen von 10 bis 70 und vorzugsweise 25 bis 35 Gew.-% enthalten sein können.

### Weitere Hilfs- und Zusatzstoffe

Bei den erfindungsgemäßen Reinigungsmittel handelt es sich um Cremes oder Lotionen, vorzugsweise in O/W-Form, wie beispielsweise Hautcremes, Haarcremes, Haarkuren, Conditionern, Duschgele oder Shampoos, aber auch Stifzubereitungen wie Lippenstifte oder Deosticks. Diese Mittel können ferner als weitere Hilfs- und Zusatzstoffe milde Tenside, Emulgatoren, Überfettungsmittel, Perlglanzwachse, Konsistenzgeber, Polymere, Siliconverbindungen, Fette, Wachse, Stabilisatoren, biogene Wirkstoffe, Deowirkstoffe, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäureatkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Als geeignete **Verdickungsmittel** sind neben den eingangs aufgeführten polymeren Verbindungen weiterhin Aerosil-Typen (hydrophile Kieselsäuren), Tenside, wie beispielsweise ethoxylierte Fettsäureglyceride, Ester von Fettsäuren mit Polyolen, wie beispielsweise Pentaerythrit oder Trimethylolpropan, Fettalkoholethoxylate mit eingeengter Homologenverteilung oder Alkyloligoglucoside sowie Elektrolyte, wie Kochsalz und Ammoniumchlorid zu verstehen.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/lsobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc. Cosm.Chem. 24, 281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)].** Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methylolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **insekten-Repellentien** kommen N,N-Diethyl-m-toluamid, 1,2-Pentandiol oder Insekten-Repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

**Beispiele 1 bis 4, Vergleichsbeispiel V1.** Zur Herstellung der erfindungsgemäßen Haarspülungen 1 bis 4 wurde jeweils eine etwa 30 Gew.-%ige Dispersion von Cetylstearylalkohol in Wasser eingesetzt, die als Stabilisatoren jeweils 1 Gew.-% - bezogen auf die Dispersion - Sodium Lauryl Sulfate (D1), Sodium Laureth Sulfate (D2) bzw. Coco Glucosides (D3,D4) enthielt. Die Herstellung der Dispersionen erfolgte nach dem Mikrodispersions-Verfahren. Die Dispersion D1 wies eine mittlere Teilchengröße von 55 µm auf, die Dispersionen D2 und D3 von 40 µm und die Dispersion D4 von 10 µm. Im Fall der Vergleichsspülung V1 wurde der Cetylstearylalkohol direkt, also nicht als wäßrige tensidische Dispersion eingerührt. Die Herstellung der fünf Spülungen erfolgte durch Homogenisieren der Komponenten bei 20°C. Die Viskosität wurde unmittelbar sowie nach Lagerung über einen Zeitraum von 4 Wochen bei 40°C nach der Brookfield-Methode bestimmt (10 Upm, Spindel 1). Die Ergebnisse sind in Tabelle 1 zusammengefaßt:

**Tabelle 1**

| **Viskosität und Stabilität von Haarspülungen - Mengenangaben als Gew.-% Aktivsubstanz** | | | | | |
|---|---|---|---|---|---|
| **Zusammensetzung / Performance** | **1** | **2** | **3** | **4** | **V1** |
| Fettalkoholdispersion D1 | 25,0 | - | - | - | - |
| Fettalkoholdispersion D2 | - | 25,0 | - | - | - |
| Fettalkoholdispersion D3 | - | - | 25,0 | 25,0 | - |
| Cetearyl Alcohol | - | - | - | - | 25,0 |
| Octyldodecanol | 2,0 | 2,0 | 2,0 | 2,0 | 2,0 |
| Coco Glucosides | 2,0 | 2,0 | 2,0 | 4,0 | 2,0 |
| Cetrimonium Chloride | 4,0 | 4,0 | 4,0 | 4,0 | 4,0 |
| Wasser | ad 100 | | | | |
| ***Viskosität [mPas]*** | | | | | |
| ***- sofort*** | 13.000 | 13.000 | 13.500 | 14.000 | 9.000 |
| ***- nach 4 w (40°C)*** | 12.000 | 12.000 | 13.000 | 13.500 | - |
| ***Stabilität nach 4 w (40°C)*** | stabil | stabil | stabil | stabil | 2phasig |
| (wobei die Dispersion D1 durch ihre Teilchen größe nicht im Rahmen der Erfindung ist). | | | | | |

| **Rezepturbeispiele (Mengenangaben als Gew.-%, Wasser ad 100 %)** | | | | | |
|---|---|---|---|---|---|
| **Haarmilch** | | | | | |
| Fettalkoholdispersion D1 | | | | 5,0 | |
| Dicocoethyl Hydroxyethylmonium Methosulfate (amd) Propylene Glycol | | | | 2,0 | |
| Coco Glucosides (and) Glyceryl Oleate | | | | 2,0 | |

| **Shampoo** | | | | | |
|---|---|---|---|---|---|
| Sodium Laureth Sulfate | | | | 11,0 | |
| Cocamidopropyl Betaine | | | | 7,0 | |
| Lauryl Glucoside | | | | 4,0 | |
| Hydrolyzed Wheat Proteine | | | | 2,0 | |
| Fettalkoholdispersion D2 | | | | 3,0 | |
| Sodium Chloride | | | | 1,6 | |
| | | | | | |

| **Pflegeshampoo** | | | | | |
|---|---|---|---|---|---|
| Sodium Laureth Sulfate 8,0 | | | | | |
| Lauryl Glucoside | | | | 4,0 | |
| Laurydimonium Hydroxypropyl Hydrolyzed Collagen | | | | 4,0 | |
| Fettalkoholdispersion D3 | | | | 3,0 | |
| Sodium Chloride | | | | | |
| | | | | | |

| **Perlglänzendes Proteinshampoo** | | | | | |
|---|---|---|---|---|---|
| Decyl Glucoside | | | | 12,3 | |
| Cocamidopropyl Betaine | | | | 3,0 | |
| Fettalkoholdispersion D1 | | | | 3,0 | |
| Sodium Laureth Sulfate | | | | 2,0 | |
| Hydrolyzed Collagen | | | | 2,0 | |
| PEG-7 Glyceryl Cocoate | | | | 2,0 | |
| PEG-3 Distearate (and) Sodium Laureth Sulfate | | | | 2,0 | |
| Polyacryl Acid | | | | 0,9 | |
| Phenoxyethanol | | | | 0,5 | |
| | | | | | |

| **Shampoo für feines und fettiges Haar** | | | | | |
|---|---|---|---|---|---|
| Sodium Laureth Sulfate | | | | 14,3 | |
| Decyl Glucoside | | | | 10,0 | |
| Cocoamidopropyl Betaine | | | | 10,0 | |
| Fettalkoholdispersion D2 | | | | 10,0 | |
| Potassium Abietoyl Hydrolyzed Collagen | | | | 5,1 | |
| Sodium Chloride | | | | 1,0 | |
| Polyquaternium-10 | | | | 0,2 | |

| **Haarspülung für strapaziertes Haar** | | | | | |
|---|---|---|---|---|---|
| Fettalkoholdispersion D3 | | | | 40,0 | |
| Cetrimonium Chloride | | | | 4,0 | |
| Octyldodecanol | | | | 2,0 | |
| Tocopheryl Acetate | | | | 2,0 | |
| | | | | | |

| **Haarspülung** | | | | | |
|---|---|---|---|---|---|
| Fettalkoholdispersion D1 | | | | 25,0 | |
| Cetrimonium Chloride | | | | 4,0 | |
| Lauryl Glucoside | | | | 2,0 | |
| Glycerin | | | | 5,0 | |
| | | | | | |

| **Haarspülung** | | | | | |
|---|---|---|---|---|---|
| Fettalkoholdispersion D2 | | | | 25,0 | |
| Dicocoylethyl Hydroxyethylmonium Methosulfate (and) Propylene Glycol | | | | 2,0 | |
| Lauryl Glucoside | | | | 2,0 | |
| Octyl Dodecanol | | | | 1,0 | |
| | | | | | |

| **Leave-on Haarkur** | | | | | |
|---|---|---|---|---|---|
| Lauryl Glucoside | | | | 0,5 | |
| Oleyl Erucate | | | | 0,5 | |
| Hydrolyzed Wheat Protein | | | | 0,8 | |
| Tocopheryl Acetate | | | | 0,2 | |
| Fettalkoholdispersion D3 | | | | 10,0 | |
| Lauryldimonium Hydroxypropyl Hydrolyzed Wheat Protein | | | | 0,8 | |
| Ethanol | | | | 10,0 | |
| | | | | | |

| **Duschbad mit Pflegeadditiv** | | | | | |
|---|---|---|---|---|---|
| Coco Glucoside | | | | 10,0 | |
| Sodium Laureth Sulfate | | | | 20,0 | |
| Disodium Laurethsulfosuccinate | | | | 5,0 | |
| Lauryl Glucoside (and) Glycol Distearate (and) Glycerine | | | | 5,0 | |
| Hydrolyzed Wheat Gluten | | | | 2,0 | |
| Fettalkoholdispersion D1 | | | | 10,0 | |

| **Duschbad & Emulsion Two-in-one** | | | | | |
|---|---|---|---|---|---|
| Fettalkoholdispersion D2 | | | | 15,0 | |
| Sodium Laureth Sulfate | | | | 40,0 | |
| Lauryl Glucoside | | | | 20,0 | |
| PEG-7 Glyceryl Cocoate | | | | 2,0 | |
| Cocamidopropyl Betaine | | | | 2,0 | |
| Sodium Styrene/Acrylates | | | | 2,0 | |
| | | | | | |

| **Leichte W/O-Lotion** | | | | | |
|---|---|---|---|---|---|
| Polyglyceryl-2 Polyhydroxystearate | | | | 3,0 | |
| Glyceryl Oleate | | | | 1,0 | |
| Zinc Stearate | | | | 1,0 | |
| Hexyl Laurate | | | | 6,0 | |
| Cetearyl Isononanoate | | | | 7,0 | |
| Dicaprylyl Ether | | | | 6,0 | |
| Hexyldecanol | | | | 3,0 | |
| Glycerin | | | | 5,0 | |
| Magnesium Sulfate | | | | 1,0 | |
| Fettalkoholdispersion D3 | | | | 3,0 | |
| | | | | | |

| **Kaltemulgierte W/O-Lotion** | | | | | |
|---|---|---|---|---|---|
| PEG-7 Hydrogenated Castor Oil | | | | 3,0 | |
| Polyglyceryl-3 Diisostearate | | | | 1,0 | |
| Zinc Stearate | | | | 1,0 | |
| Hexyl Laurate | | | | 6,0 | |
| Cetearyl Isononanoate | | | | 7,0 | |
| Dicaprylyl Ether | | | | 6,0 | |
| Hexyldecanol | | | | 3,0 | |
| Glycerin | | | | 5,0 | |
| Magnesium Sulfate | | | | 1,0 | |
| Fettalkoholdispersion D1 | | | | 5,0 | |
| | | | | | |

| **O/W-Sonnenschutzlotion** | | | | | |
|---|---|---|---|---|---|
| Sodium Laureth Sulfate | | | | 4,0 | |
| Fettalkoholdispersion D2 | | | | 25,0 | |
| Coco Caprylate/Caprate | | | | 3,0 | |
| Cetearyl Isononanoate | | | | 3,0 | |
| Tocopheryl Acetate | | | | 1,0 | |
| Octyl Methoxycinnamate | | | | 7,5 | |
| Butyl Methoxybenzoylmethane | | | | 2,5 | |
| Benzophenone-11 | | | | 1,0 | |
| Glycerine | | | | 3,0 | |
| Soluble Collagen | | | | 2,0 | |
| | | | | | |

| **Flüssige O/W-Emulsion** | | | | | |
|---|---|---|---|---|---|
| Fettalkoholdispersion D3 | | | | 10,0 | |
| Dicocoylethyl Hydroxyethylmonium Methosulfate (and) Propylene Glycol | | | | 1,0 | |
| Dicaprylyl Ether | | | | 2,0 | |
| Octyldodecanol | | | | 4,0 | |
| Glycolic Acid | | | | 0,3 | |
| DL-Malic Acid | | | | 0,15 | |
| L-Tartaric Acid | | | | 0,15 | |
| Citric Acid | | | | 0,1 | |
| Lactic Acid | | | | 0,3 | |
| DL-Sodium Lactate | | | | 1,0 | |
| Sodium Pyruvate | | | | 0,3 | |
| Glycerin | | | | 3,0 | |

## Patentansprüche

1. Verfahren zur Herstellung von kosmetischen Reinigungsmitteln mit erhöhter Viskosität, bei dem man wäßrige tensidische Fettalkoholdispersionen, die Fettalkohole in Mengen von 5 bis 50 Gew.-% - bezogen auf die Dispersionen - enthalten und eine Teilchengröße kleiner 50 µm aufweisen, mit Ölkomponenten kalt verrührt.

2. Verfahren nach Anspruch 1, **dadurch gekennzeichnet, daß** man Dispersionen einsetzt, die Fettalkohole der Formel (I) enthalten,
**R**^{**1**}**OH ( I)**
in der R¹ für einen linearen oder verzweigten, gesättigten oder ungesättigten Alkylrest mit 6 bis 30 Kohlenstoffatomen steht.

3. Verfahren nach den Ansprüchen 1 und 2, **dadurch gekennzeichnet, daß** man Dispersionen einsetzt, die anionische, nichtionische, kationische und/oder amphotere bzw. zwitterionische Tenside enthalten.

4. Verfahren nach den Ansprüchen 1 bis 3, **dadurch gekennzeichnet, daß** man Dispersionen einsetzt, die die Tenside in Mengen von 0,1 bis 10 Gew.-% - bezogen auf die Dispersionen enthalten.

5. Verfahren nach den Ansprüchen 1 bis 4, **dadurch gekennzeichnet, daß** man Ölkomponenten einsetzt, die ausgewählt sind aus der Gruppe, die gebildet wird von Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primären Alkoholen, substituierten Cyclohexanen, linearen C₆-C₂₂-Fettalkoholcarbonaten, Guerbetcarbonaten, Estern der Benzoesäure mit linearen und/oder verzweigten C₆-C₂₂-Alkoholen, Dialkylethern, Ringöffnungsprodukten von epoxidierten Fettsäureestern mit Polyolen, Siliconölen und/oder aliphatischen bzw. naphthenischen Kohlenwasserstoffen.

6. Verfahren nach den Ansprüchen 1 bis 3, **dadurch gekennzeichnet, daß** man polymere Verdickungsmittel einsetzt.

## Claims

1. A process for the production of high-viscosity cosmetic cleansing compositions, wherein aqueous surfactant-containing fatty alcohol dispersions, which contain fatty alcohols in quantities of 5 to 50% by weight, based on the dispersions, and have a particle size below 50 µm, are cold-stirred with oil components.

2. A process as claimed in claim 1, **characterized in that** dispersions containing fatty alcohols corresponding to formula (I):
**R**^{**1**}**OH (I)**
in which R¹ is a linear or branched, saturated or unsaturated alkyl group containing 6 to 30 carbon atoms, are used.

3. A process as claimed in claims 1 and 2, **characterized in that** dispersions containing anionic, nonionic, cationic and/or amphoteric or zwitterionic surfactants are used.

4. A process as claimed in claims 1 to 3, **characterized in that** dispersions containing the surfactants in quantities of 0.1 to 10% by weight, based on the dispersions, are used.

5. A process as claimed in claims 1 to 4, **characterized in that** oil components selected from the group consisting of Guerbet alcohols based on fatty alcohols containing 6 to 18 carbon atoms, esters of linear C₆₋₂₂ fatty acids with linear C₆₋₂₂ fatty alcohols, esters of branched C_{6ν13} carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, esters of C₂₋₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, ring opening products of epoxidized fatty acid esters with polyols, silicone oils and/or aliphatic or naphthenic hydrocarbons are used.

6. A process as claimed in claims 1 to 3, **characterized in that** polymeric thickeners are used.

## Revendications

1. Procédé pour la fabrication de compositions cosmétiques de nettoyage à haute viscosité, dans lequel on délaie à froid des dispersions aqueuses d'alcools gras, contenant des tensioactifs, qui contiennent des alcools gras en quantités de 5 à 50 % en poids - par rapport aux dispersions - et ont une taille de particule inférleure à 50 µm, avec des composants huileux.

2. Procédé selon la revendication 1,
**caractérisé en ce qu'**
on utilise des dispersions qui contiennent des alcools gras de formule (I)
R¹OH (I)
dans laquelle R¹ représente un radical allyle linéaire ou ramifié, saturé ou insaturé, ayant de 6 à 30 atomes de carbone.

3. Procédé selon les revendications 1 et 2,
**caractérisé en ce qu'**
on utilise des dispersions qui contiennent des tensioactifs anioniques, non ioniques, canoniques et/ou amphotères ou zwitterioniques.

4. Procédé selon les revendications 1 à 3,
**caractérisé en ce qu'**
on utilise des dispersions qui contiennent les tensioactifs en quantités de 0,1 à 10 % en poids, par rapport aux dispersions.

5. Procédé selon les revendications 1 à 4,
**caractérisé en ce qu'**
on utilise des composants huileux qui sont choisis dans le groupe qui est constitué par des alcools de Guerbet à base d'alcools gras ayant de 6 à 18 atomes de carbone, des esters d'acides gras linéaires en C₆-C₂₂ avec des alcools gras en C₆-C₂₂ linéaires, des esters d'acides carboxyliques en C₆-C₁₃ ramifiés avec des alcools gras en C₆-C₂₂ linéaires, des esters d'acides gras linéaires en C₆-C₂₂ avec des alcools ramifiés, des esters d'acides gras linéaires et/ou ramifiés avec des alcools polyhydroxylés et/ou des alcools de Guerbet, des triglycérides à base d'acides gras en C₆-C₁₀, des mélanges liquidas de mono-/di-/triglycérides à base d'acides gras en C₆-C₁₈, des esters d'alcools gras en C₆-C₂₂ et/ou d'alcools de Guerbet avec des acides carboxyliques aromatiques, des esters d'acides dicarboxyliques en C₂-C₁₂ avec des alcools linéaires ou ramifiés ayant de 1 à 22 atomes de carbone ou des polyols ayant de 2 à 10 atomes de carbone et 2 à 6 groupes hydroxy, des huiles végétales, des alcools primaires ramifiés, des cyclohexanes substitués, des carbonates d'alcools gras en C₆-C₂₂ linéaires, des carbonates de Guerbet, des esters de l'acide benzoïque avec des alcools en C₆-C₂₂ linéaires et/ou ramifiés, des éthers de dialkyle, des produits d'ouverture de cycle d'esters d'acides gras époxydés avec des polyols, des huiles de silicone et/ou des hydrocarbures aliphatiques ou naphténiques.

6. Procédé selon les revendications 1 à 3,
**caractérisé en ce qu'**
on utilise des épaississants polymères.
